# EUROPEAN PATENT APPLICATION

(11) **EP 1 508 283 A1**
(43) Date of publication of application: **23.02.2005**
(21) Application number: 03752924.5
(22) Date of filing: 20.05.2003
(51) Int. Cl.: A41B 9/02

(54) **DISPOSABLE SHORT PANTIES AND METHOD FOR PRODUCING THE SAME**

(30) Priority: 22.05.2002 JP 2002147755
(71) Applicant: UNI-CHARM CORPORATION, Kawanoe-shi Ehime 799-0111 (JP)
(72) Inventor: SAKAGUCHI, Satoru Technical Center, Uni-Charm Corp, Mitoyo-gun, Kagawa 769-1602 (JP); ISHIKAWA, Hiroki Technical Center, Uni-Charm Corp., Mitoyo-gun, Kagawa 769-1602 (JP)
(74) Representative: Fitchett, Stuart Paul
(86) International application number: PCT/JP2003/006290
(87) International publication number: WO 2003/096828

(57) **Abstract**

Disposable undergarment comprising a sheet body constituting an enclosure, an elastic member for expanding/shrinking a part of the sheet body, and a pair of leg openings for passing the legs of a wearer. The sheet body comprises an outer face sheet, and inner face sheet having the shape and size substantially identical to those of the outer face sheet. The elastic member is stretched between the outer and inner sheets while being elongated previously along the outer circumferential fringe of the sheet body, and the pair of leg openings are provided at positions on the outer side of the central area of the sheet body in the vicinity of the inside of the elastic body. Disposable undergarment having no joint around the waist area in which the elastic body provided to the sheet body shrinks to form a waist opening are thereby obtained.

## Description

### FIELD OF THE INVENTION

The invention relates to a disposable undergarment, and more particularly, to an undergarment having no cemented section in a girth region.

### RELATED ART

As described in JP-UM-B-3-4260, the following structure has been proposed for a conventional disposable undergarment. Namely, a front abdominal sheet―which is formed from disposable material and on which a front abdominal piece and a crotch piece are provided adjacently―and a rear abdominal sheet on which a rear abdominal piece and the crotch piece provided adjacently are sewn together at a front abdominal region, a rear abdominal region, and a crotch section. During sewing operation, a body fluid absorbent and a liquid-impervious sheet are sewed onto the crotch section.

The conventional disposable undergarment is discarded without being laundered. Particularly, the disposable undergarment is suitable for carrying and changing during outdoor activities or travel. However, the front abdominal sheet and the rear abdominal sheet, both being formed from disposable material, are sewn together at the front abdominal region, the rear abdominal region, and the crotch section. Therefore, the thus-cemented sections are present in the front and rear abdominal regions and the crotch section. Since the materials of the undergarment are in a superposed relation in the cemented section, the cemented section is voluminous. When the undergarment is folded, the cemented sections obstruct folding of the undergarment, making it difficult to render the undergarment compact. Further, the cemented sections appear through a garment such as a pair of trousers or a skirt when the garment is worn over the undergarment, thereby impairing a silhouette of the garment.

### DISCLOSURE OF THE INVENTION

The invention has been conceived in view of the foregoing problem and provides a disposable undergarment which can be folded compact and do not affect a silhouette of a garment, such as a pair of trousers, when the garment is worn over the undergarment.

According to the invention, a disposable undergarment having a waist opening section and no cemented section in a girth has been successfully produced by placing a previously-stretched elastic member along an outer peripheral edge and in a waist opening section of a sheet member constituting a disposable undergarment, wherein the waist opening section is formed as a result of the thus-stretched elastic member having undergone contraction. Thus, the object has been achieved.

More specifically, the elastic member is placed so as to draw a two lines consisting of waveform like curves or straight lines such that the two waveform like curves or straight lines coming into close proximity to each other or crossing each other, the elastic member becomes substantially continuous at the time of formation of an undergarment. Hence, the inventors have discovered that a waist opening section and leg opening sections can be formed without sewing together a front abdominal section, a rear abdominal section, and a crotch section, which would otherwise be required by a conventional disposable undergarment. By means of this finding, the present invention has been completed.

Further, the disposable undergarment of the invention has been provided on the basis of the novel idea that the previously-stretched elastic member is placed in a stretched manner on a traveling sheet so as to draw two waveform like curves or straight lines such that the these two waveform like curves or straight lines come into proximity to each other or cross each other.

More specifically, the invention provides the following:
(1) A disposable undergarment having a sheet member to constitute an exterior, an elastic member for causing a portion of the sheet member to contract, and a pair of leg opening sections through which a wearer's legs penetrate, wherein the sheet member is formed from a material which is seamless with respect to a circumferential direction of the leg when the sheet member is worn.
   According to the invention (1), the sheet member constituting an exterior, is formed from a material which is seamless with respect to a circumferential direction of the leg when the sheet member is worn. Therefore, there is obviated a necessity for sewing together the front abdominal section, the rear abdominal section, and the crotch section, all pertaining to the sheet member, which has been required for a conventional disposable undergarment. Hence, the resultant undergarment has no cemented section in the girth.
(2) The disposable undergarment according to (1), wherein the elastic member is provided along an substantially outer peripheral edge of the sheet member in a previously-stretched manner, the pair of leg opening sections are formed in the vicinity of the inside of the elastic member and at positions outside the center of the sheet member; and wherein the waist opening section is formed as a result of the elastic member stretched in the sheet member undergoing contraction.
   According to the invention (2), the elastic member is provided along an substantially outer peripheral edge of the sheet member in a previously-stretched manner. Since the elastic member is provided in a previously-stretched state, the outer peripheral edge of the sheet member is pulled and contracts when the elastic member has undergone contraction. As a result, a ring-shaped waist opening section is formed, whereby an undergarment is formed. Consequently, no cemented section is provided in the direction of a girth or other directions.
(3) The disposable undergarment according to (1) or (2), wherein the sheet member is formed from an outer surface sheet and an inner surface sheet which is substantially identical in size and shape with the outer surface sheet, the elastic member is interposed between the outer and inner surface sheets and along an outer peripheral edge of the sheet member in a previously-stretched state, the pair of leg opening sections are provided in the vicinity of the inside of the elastic member and at positions outside the center of the sheet member; and wherein a waist opening section is formed as a result of the elastic member stretched on the sheet member having undergone contraction.
   According to the invention (3), the sheet member is formed from the outer surface sheet and the inner surface sheet, and the elastic member is interposed between the outer and inner surface sheets and along an outer peripheral edge of the sheet member while having been stretched beforehand. Since the elastic member is provided in a previously-stretched state, the outer peripheral edge of the sheet member is pulled and contracts when the elastic member has undergone contraction. As a result, a ring-shaped waist opening section is formed, whereby an undergarment is formed. Consequently, no cemented section is provided around the girth.
(4) The disposable undergarment according to any one of (1) through (3), wherein a plurality of the elastic members are provided around the waist opening section and a girth in a previously-stretched state.
   According to the invention (4), the elastic members are provided along the waist opening section and the girth while having been stretched beforehand. Since the elastic members are provided in a previously-stretched state, the outer peripheral edge of the sheet member is pulled and contracts when the elastic members have undergone contraction. As a result, a ring-shaped waist opening section is formed, and the waist opening section and the girth become elastic. Thus, the undergarment can be worn so as to conform to the shape of the body.
   Here, the term "waist opening section" means a portion of the diaper which conforms to a position on the body above the hip bone. Further, the term "girth section" means a part of the diaper which conforms to the area of the body extending from the hip bone to the crotch.
(5) The disposable undergarment according to (4), wherein the elastic members provided in the waist opening section cross each other in either front and rear abdominal regions or right and left abdominal regions.
   According to the invention (5), the elastic members provided in the waist opening section are laid such that the elastic members cross each other in either front and rear abdominal regions or right and left abdominal regions. Therefore, the elastic members become continuous at intersection positions. As a result of the continuous elastic members undergoing contraction, a ring-shaped waist opening section is formed, thereby producing an undergarment having no cemented section. Even when the intersections between the elastic members are located in the front and rear abdominal regions or the right and left abdominal regions, the elastic members are stretched along the outer peripheral edge of the sheet member. Hence, a ring-shaped waist opening section is formed in a similar manner, and an undergarment having no cemented section can be obtained.
   Here, the term "front abdominal region" means a portion of a brief-type diaper facing the abdomen of the body (more specifically, the abdomen of the body which is brought into contact upon wearing them). The term "rear abdominal region" means a portion of the diaper facing the back of the body more specifically, the back of the body which is brought into contact upon wearing them). The term "right abdominal region" means a portion of the diaper facing the right side of the body (more specifically, the right side of the body which is brought into contact upon wearing them). The term "left abdominal region" means a portion of the diaper facing the left side of the body (more specifically, left side of the body which is brought into contact upon wearing them). In addition, "cross" means to describe a straight lines crossing each other including the two or more straight line crossing at a point, each individual straight lines crossing with each other, as well as straight lines brought into contact with each other.
(6) The disposable undergarment according to (4), wherein the elastic members provided in the waist opening section cross or overlap each other at one point each in either front and rear abdominal regions or right and left abdominal regions.
   According to the invention (6), the elastic members provided in the waist opening section are laid such that the elastic members cross or overlap each other at one point each in either front and rear abdominal regions or right and left abdominal regions. Therefore, the elastic members become continuous at intersection positions. As a result of the continuous elastic members undergoing contraction, a ring-shaped waist opening section is formed, thereby producing an undergarment having no cemented section. Even when the intersections between the elastic members are located in the front and rear abdominal regions or the right and left abdominal regions, the elastic members are stretched along the outer peripheral edge of the sheet member. Hence, a ring-shaped waist opening section is formed in a similar manner, and an undergarment having no cemented section can be obtained.
(7) The disposable undergarment according to any one of (1) through (6), wherein the elastic member is rubber yarn, flat rubber, or a ribbon-shaped elastic body.
   According to the invention (7), the elastic member is rubber yarn, flat rubber, or a ribbon-shaped elastic body. Since the elastic member is extendable, the elastic member undergoes contraction when released, so long as the elastic member has been provided while having been stretched beforehand. The outer peripheral edge of the sheet member is pulled by the elastic member, thereby forming a ring-shaped waist opening section. Thus, an undergarment having no cemented section is produced.
(8) The disposable undergarment according to any one of (1) through (7), wherein the sheet member is hydrophobic and gas-permeable nonwoven fabric cloth.
   According to the invention (8), the sheet member is hydrophobic and gas-permeable nonwoven fabric cloth. Therefore, when having worn the undergarment, the wearer has pleasant texture. Further, since the undergarment possesses moisture permeability, the wearer does not suffer from humidity.
(9) The disposable undergarment according to (8), wherein the sheet member is nonwoven fabric cloth provided with a deodorant.
   According to the invention (9), the sheet member is nonwoven fabric cloth provided with a deodorant. Therefore, even when the undergarment is stained with body fluids, such as urine, the undergarment is deodorized. Unpleasant scent, which would otherwise arise when the undergarment is discarded after use, does not arise, and hence the undergarment is easy to handle.
(10) The disposable undergarment according to (8), wherein the sheet member is nonwoven fabric cloth provided with a fragrance.
   According to the invention (10), the sheet member is nonwoven fabric cloth provided with a fragrance. Even when the undergarment has become stained with body fluids, such as urine, the scent is dissolved by the fragrance. Hence, unpleasant scent, which would otherwise arise when the undergarment is discarded after use, does not arise, and hence the undergarment is easy to handle.
(11) The disposable undergarment according to any one of (8) through (10), wherein the nonwoven fabric cloth is nonwoven fabric cloth selected from the group consisting of continuous polyolefine-based resin fibers.
   According to the invention (11), the nonwoven fabric cloth is nonwoven fabric cloth selected from the group consisting of continuous polyolefine-based resin fibers. The nonwoven fabric cloth has hydrophobic, gas-permeable characteristics, softness, and high strength and is comparatively inexpensive. When having worn the undergarment, the wearer has good texture. Further, the undergarment has gas permeable characteristics, and hence moisture is not accumulated in the undergarment, thereby preventing excess body moisture. The nonwoven fabric cloth includes spun bond nonwoven fabric cloth made of polypropylene or the like; nonwoven fabric cloth made of a spun bond layer and a melt blown layer; and a spun bond nonwoven fabric cloth formed from polypropylene and polyethylene.
(12) The disposable undergarment according to any one of (8) through (11), wherein a crotch section of the sheet member is liquid-impervious and moisture-permeable.
   According to the disposable undergarment of the invention (12), the crotch section of the sheet member is liquid-impervious and moisture-permeable. Therefore, even when body fluids, such as menstrual blood, have leaked, there is no chance of the body fluids seeping. Further, no moisture is accumulated in the undergarment. In order to impart liquid-impervious and moisture-pervious characteristics to the crotch section of the sheet member, a sheet, such as a liquid-impervious and moisture-pervious film, can be inserted into the crotch section of the sheet member, or liquid-impervious and moisture-pervious resin can be laminated. Moreover, a comparatively thin absorbent layer, such as tissue paper, hydrophilic nonwoven fabric cloth, or air-raid pulp, may also be provided on the surface of the liquid-impervious layer in the crotch section, the surface facing the wearer. As a result, leakage of urine stemming from incontinence or leakage of body fluids, such as menstrual blood, can be absorbed.
(13) The disposable undergarment according to any one of (8) through (11), wherein the undergarment is to be used for a sanitary product having a mechanical fastener or for attaching an auxiliary product of the sanitary product.
   According to the invention (13), the sheet member is formed from nonwoven fabric cloth, and hence the mechanical faster is likely to attach to the sheet member. Hence, a mechanical fastener is provided in the crotch section, thereby facilitating attachment of a sanitary product, such as a labia-pad or an incontinence pad, or an auxiliary product, to the undergarment. Thus, the undergarment can be utilized as a sanitary undergarment.
(14) The disposable undergarment according to any one of (1) through (11), wherein the undergarment is to be used as a lining of an ordinary underwear, to thereby prevent staining of the underwear.
   According to the invention (14), no cemented section is provided such that the undergarment is not voluminous and is suitable for use as an inner lining of ordinary underwear upon wearing the disposable undergarment.
(15) A method for manufacturing a disposable undergarment, comprising at least an elastic-member-equipped continuous sheet member manufacturing step of manufacturing a continuous sheet member having an elastic member by continuously placing an elastic member on a continuously-traveling outer surface sheet in a stretched manner, a continuous sheet member shaping step of forming leg opening sections in areas of the continuous sheet member where the elastic member is not provided and shaping the outer shape of the sheet member by trimming and an undergarment separation step of separating pieces of disposable undergarments by cutting the continuous sheet member in transverse direction.

According to the invention (15), a continuous sheet member having an elastic member is continuously placed on a continuously-traveling outer surface sheet in a stretched manner. Leg opening sections are formed in areas of the continuous sheet member where the elastic member is not provided, and the outer shape of the continuous sheet member is shaped. Subsequently, the shaped continuous sheet member is cut in a transverse direction thereof, thereby producing pieces of undergarments.

The elastic member is arranged continuously along the outer peripheral edge of the shaped continuous sheet member. Hence, when the continuous sheet member having the elastic member provided thereon is cut, separates into individual sheet member, thus the extended elastic member undergoes contraction, thereby forming a ring-shaped waist opening section and become a undergarment. Accordingly, processes for sewing a front abdominal section, a rear abdominal section, and a crotch section becomes obviated, and hence productivity is improved.

### BRIEF DESCRIPTION OF THE DRAWINGS

Fig. 1 is a perspective view showing a first embodiment of a disposable undergarment of the invention;
Fig. 2A is a developed plan view showing a developed state of the first embodiment of the disposable undergarment of the invention
Fig. 2B is a cross-sectional view taken along line X-X' shown in Fig. 2A;
Figs. 3A is a fragmentary plan views showing first carried out example on the flow of elastic members provided on a sheet member in a stretched manner
Figs. 3B is a fragmentary plan views showing second carried out example on the flow of elastic members provided on a sheet member in a stretched manner
Figs. 3C is a fragmentary plan views showing third carried out example on the flow of elastic members provided on a sheet member in a stretched manner
Figs. 3D is a fragmentary plan views showing fourth carried out example on the flow of elastic members provided on a sheet member in a stretched manner
Figs. 3E is a fragmentary plan views showing fifth carried out example on the flow of elastic members provided on a sheet member in a stretched manner
Fig. 4A is a developed view of a modification of the first embodiment shown in Figs. 2A
Fig. 4B is a cross-sectional view taken along line X-X' shown in Fig. 4A;
Fig. 5A is a developed plan view showing a developed state of a second embodiment of the disposable undergarment of the invention
Fig. 5B is a cross-sectional view taken along line X-X' shown in Fig. 5A;
Fig. 6A is a developed plan view showing a developed state of a third embodiment of the disposable undergarment of the invention
Fig. 6B is a cross-sectional view taken along line X-X' shown in Fig. 6A;
Fig. 7 is a plan view showing the flow of elastic members for manufacturing the disposable undergarment of the invention.

### DESCRIPTION OF THE PREFERRED EMBODIMENT

Embodiments of the invention will now be described by reference to the drawings.

### [First Embodiment]

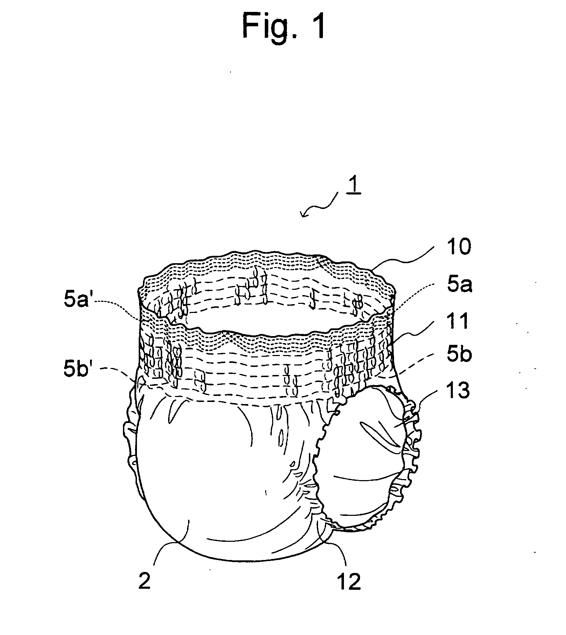

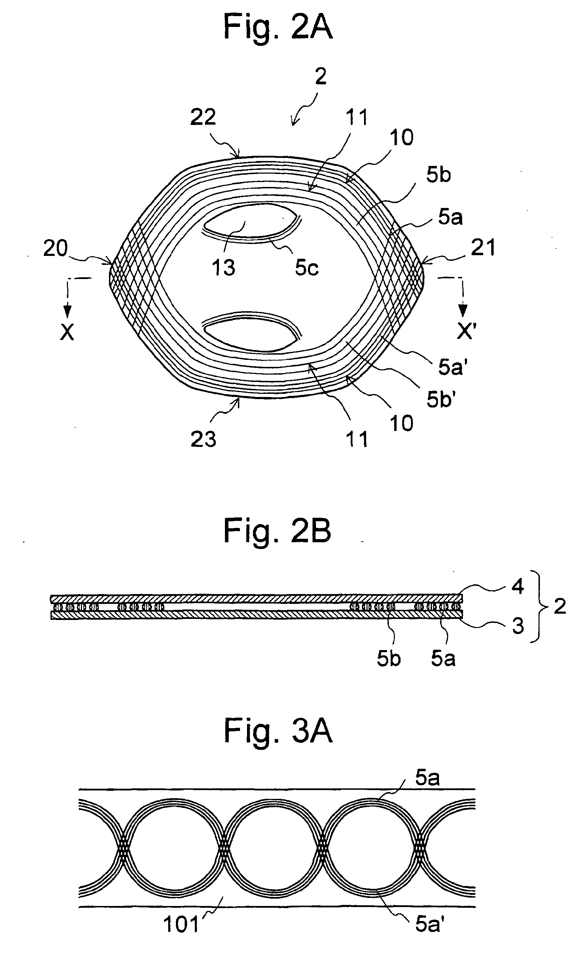

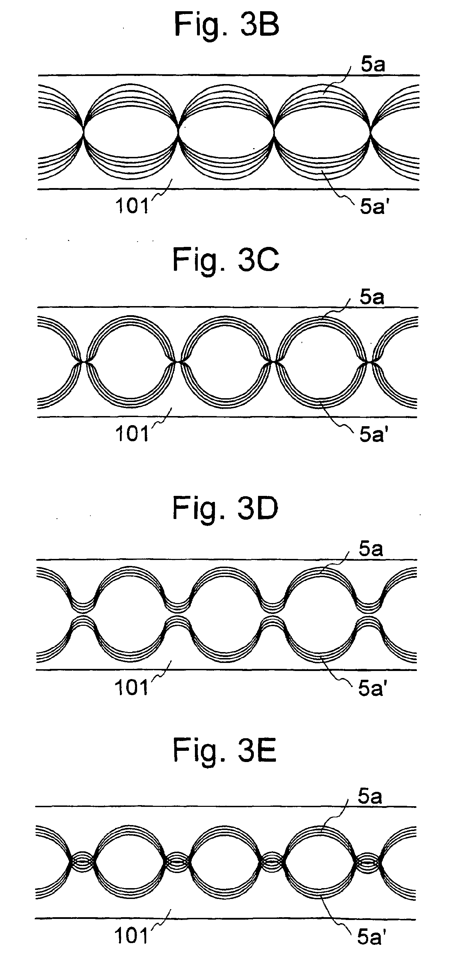

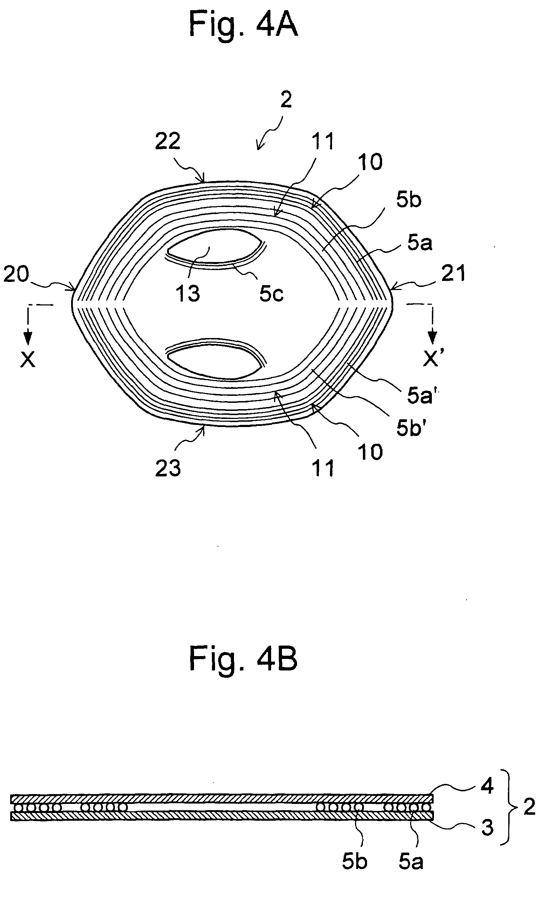

Fig. 1 is a perspective view showing a first embodiment of a disposable undergarment of the invention; Fig. 2A is a developed plan view showing a developed state of the first embodiment of the disposable undergarment of the invention; Fig. 2B is a cross-sectional view taken along line X-X' shown in Fig. 2A; Figs. 3A is a fragmentary plan views showing first carried out example on the flow of elastic members provided on a sheet member in a stretched manner; Figs. 3B is a fragmentary plan views showing second carried out example on the flow of elastic members provided on a sheet member in a stretched manner; Figs. 3C is a fragmentary plan views showing third carried out example on the flow of elastic members provided on a sheet member in a stretched manner; Figs. 3D is a fragmentary plan views showing fourth carried out example on the flow of elastic members provided on a sheet member in a stretched manner; Figs. 3E is a fragmentary plan views showing fifth carried out example on the flow of elastic members provided on a sheet member in a stretched manner; Fig. 4A is a developed view of a modification of the first embodiment shown in Figs. 2A; and Fig. 4B is a cross-sectional view taken along line X-X' shown in Fig. 4A.

As shown in Fig. 1, a disposable undergarment 1 of the embodiment comprises a waist opening section 10 for a wearer, a girth section 11, and leg sections 12, all being formed from an elasticized sheet member 2. In order to visualize a specific structure easy to understand, Fig. 2A provides a developed plan view showing a developed state of the disposable undergarment 1 when a waist opening section 10 is broadened. As shown in Fig. 2A and 2B, the sheet member 2 constituting the disposable undergarment 1 of the embodiment assumes a substantially-circular shape and comprises an outer surface sheet 3; an inner surface sheet 4; and elastic members 5a, 5a' for a waist opening section 10 and elastic members 5b, 5b' of the girth 11, both being provided between the sheets 3, 4 in a stretched manner. More specifically, in relation to the elastic members 5a, 5a', 5b, 5b'provided in a stretched manner between the outer surface sheet 3 and the inner surface sheet 4, in order to make the waist opening 10 and girth 11 sections elastic, the plurality of elastic members 5a are fixedly bonded to the sheet member 2 in a previously-stretched state across the entire right abdominal region 22 of the waist opening section 10 so as to follow an outer peripheral edge of the sheet member 2. Further, a plurality of elastic members 5a' are fixedly bonded to the sheet member 2 in a previously-stretched manner across the entire left abdominal region 23. Thus, the elastic members 5a, 5a' constitute a waist gather. Moreover, the plurality of elastic members 5b are provided across the entire right abdominal region 22 of the girth section 11 in a previously-stretched manner, and a plurality of elastic members 5b' are provided across the entire left abdominal region 23 of the girth section 11 in a previously-stretched manner. Thus, the elastic members 5b, 5b' constitute the waist gather and a girth gather, respectively.

In the embodiment, the sheet member 2 is formed from an outer surface sheet 3 and the inner surface sheet 4. The elastic members 5a, 5a' located in the waist opening section 10 and the elastic members 5b, 5b' located in the girth section 11 are interposed between the sheets 3, 4 in a stretched manner. It may be the case that only the outer surface sheet 3 is employed, or a swath-shaped elastic member may be provided on the surface of the outer surface sheet 3 and along an outer peripheral edge thereof in a stretched manner.

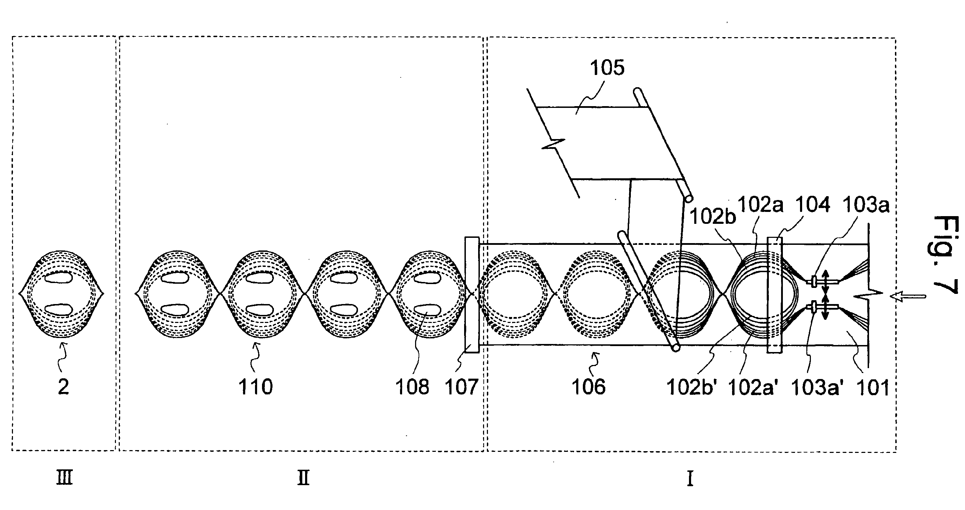

As shown in Fig. 3A, the sheet member 2 is formed from the plurality of elastic members 5a provided along the waist opening section 10 across the entire right abdominal region 22, the plurality of elastic members 5a' provided along the waist opening section 10 across the entire left abdominal region 23, are laid in a stretched manner on the continuously-supplied outer surface sheet member 101 so as to assume substantially-sinusoidal waveforms and such that the elastic members cross each other one by one and the waveforms become tied together. The inner surface continuous sheet member 105 is laid integrally on the surface of the continuous outer surface sheet member 101. Next, the sheet member 2 is cut into pieces along areas where the elastic members 5a, 5a' cross each other (See Fig.7). At this time, the elastic members 5a, 5a' are provided in a previously-stretched manner. As a result, the elastic members 5a, 5a' cross each other and are connected together at a substantially-center of front abdominal region 20 (an abdominal section) and a substantially-center of rear abdominal region 21 (a back section), and thus continue along the outer peripheral edges of the sheet member 2. Hence, when the elastic members 5a, 5a' provided along the waist opening section 10 in a stretched manner are subjected to contraction, the outer peripheral edges of the sheet member 2 are pulled, thereby constituting a continuous ring-shaped waist opening section 10.

In the embodiment, the elastic members 5a, 5a' provided along the waist opening section 10 crossing each other and become tied together. However, the invention is not limited to the embodiment. As shown in Fig. 3B, this may also be achieved by laying the elastic members 5a, 5a' on the continuously-supplied continuous outer surface sheet member 101 such that two curves cross each other at a point, or by laying the elastic members 5a, 5a' in a stretched manner on the continuously-supplied continuous outer surface sheet member 101 such that two curves come into contact with each other at valleys and peaks as shown in Fig. 3C.

In the embodiment, the elastic members 5a, 5a' of the waist opening section 10 are joined together while remaining in a crossing state. However, the invention is not limited to such a geometry. As shown in Fig. 4A and 4B, the curves may be partially separated to be discontinuous. More specifically, when the elastic members 5a, 5a' located in the waist opening section 10 are continuously arranged in a stretched manner and fixedly bonded to the sheet member 2, intersecting portions of the elastic members 5a, 5a' remain non-bonded. When the continuous sheet member is cut along the intersecting portions into disposable undergarments, the elastic members 5a, 5a' may be subjected to contraction and be slightly separated from each other at locations where the elastic members 5a, 5a' are not fixedly bonded together. In this case, the elastic members 5a, 5a' are partially discontinuous. However, the elastic members 5a, 5a' are laid along the outer peripheral edge of the sheet member 2 in a stretched and substantially continuous fashion. Hence, when the elastic members 5a, 5a' provided along the waist opening section 10 undergo contraction, forms a ring-shaped waist opening section 10 and become the undergarment. In this case, intersections of elastic member 5a, 5a' disappear, and hence, the thickness of the elastic members can be reduced as compared with the thickness of the elastic members achieved when intersections are present. Thus, the wearer does not have unusual feeling. As shown in Fig.3D, it may also achieved by drawing two waveform patterns, such that a peak of one waveform comes into contact with a valley of the other waveform, is stretched on the continuously supplied outer surface sheet 3.

In the embodiment, the outer surface sheet 3 has a substantially-circular outer shape, the elastic members are provided in a waveform pattern such as a substantially-sinusoidal shape along the circular profile. However, the pattern of the elastic members is not limited to such a waveform pattern. The curves may assume any shape, so long as the curves assume curves such as those shown in Figs. 3A to 3E or to follow the outer shape of the outer layer sheet 3. For instance, when the outer layer sheet 3 assumes a polygonal shape, the curves may assume a shape resembling a kinked graph matching the polygonal shape. In the embodiment, shape of the outer surface sheet 3 has a substantially-circular shape, such that the waveform patterns is formed along the circular profile. The curves may assume any shape, so long as the curves follow the outer shape of the outer layer sheet 3.

The elastic members 5b, 5b' provided in the girth section 11 are stretched in the substantial center of the front abdominal region 20 (abdominal section) and the substantial center of the rear abdominal region 21 (back section), respectively, such that the elastic members 5b, 5b' cross each other one by one. However, the elastic members 5b, 5b' are arranged and fixedly bonded in the same manner in which the elastic members 5a, 5a' are arranged and bonded in the waist opening section 10. The elastic members 5b, 5b' are connected such that the elastic members cross each other one by one in the same manner as mentioned previously. However, the way to connect the elastic members 5b, 5b' is not limited to this method. As in the case of the elastic members 5a, 5a' of the waist opening section 10, the elastic members 5b, 5b' may be stretched such that the elastic members cross each other one by one or such that the elastic members 5b, 5b' come into contact with or in close proximity to each other. Moreover, as in the case of the elastic members 5a, 5a' of the waist opening section 10, intersections between the elastic members 5b, 5b' may remain non-bonded and discontinuous.

Up to this point, a description has been given of a case where the elastic members 5a, 5a', 5b, 5b' cross each other at intermediate section on the front and rear abdominal regions 20, 21', which may be crossed each other respectively at the center of right and left abdominal regions 22, 23. Even in this case, the sheet member 2 assumes a substantially-circular outer shape. Hence, a disposable undergarment similar to that achieved in the embodiment is formed.

The elastic members 5a, 5a' to be provided along the waist opening section 10 of the sheet member 2 and the elastic members 5b, 5b' to be provided along the girth section 11 of the same may preferably be stretched so as to differ from each other in terms of degree of extension and stress. Specifically, the elastic members 5a, 5a' to be provided along the waist opening section 10 may preferably be stretched with a higher degree of extension and increased stress. Further, the elastic members 5b, 5b' to be provided along the girth section 11 of the sheet member 2 may preferably be stretched with a low degree of extension and small stress.

As mentioned above, the elastic members 5a, 5a', 5b, 5b' are extended such that the degree of extension and stress change from the waist opening section 10 to the girth 11, thereby enabling the undergarment to conform more closely to the shape of the body. Specifically, the waist opening section 10 of the undergarment can be fastened to such an extent that an undergarment does not slip down when being worn and conforms to the wearer's body. Since the girth 11 can gently conform to the wearer's body, stretching the elastic members is preferable. A method for changing the fit of the waist opening section 10 of the undergarment and that of the girth 11 may also be achieved by selecting the number of elastic members, intervals at which the elastic members are to be stretched, the thickness of the elastic members, and materials of the elastic members, as required, rather than changing the degree of extension of the elastic members.

In the embodiment, the elastic members to be provided along the waist opening section 10 and those to be provided along the girth 11 are set so as to differ from each other in terms of the degree of extension and stress. However, the same degree of extension or the same stress may also be employed.

A pair of leg opening sections 13 are formed in the sheet member 2. A plurality of elastic members 5c to be used for forming a leg gather are provided in a stretched manner along side edge portions within a crotch region between the leg opening sections 13. Adoption of the construction is effective for enhancing adhesion of the leg gather to the neighborhood of the leg.

The elastic members 5a, 5a', 5b, 5b' are not limited to any particular material but are formed from any material, so long as the material possesses elasticity; for example, rubber yarn made of natural/synthetic rubber or polyurethane, flat rubber, a ribbon-shaped elastic body, heat-shrinkable material, and water-absorptive shrinkable fibers.

The outer surface sheet 3 and the inner surface sheet 4, both constituting the sheet member 2, are made of material which is commonly used for a disposable undergarment, such as paper or nonwoven fabric cloth made of synthetic resin and which has flexibility, pleasant texture, and moisture permeability. Particularly, nonwoven fabric cloth is suitable in terms of softness, pleasant texture, and strength. More preferably, nonwoven fabric cloth possessing hydrophobic and gas-permeable characteristics is employed. For example, the nonwoven fabric cloth includes spun bond nonwoven fabric cloth made of polypropylene or the like; nonwoven fabric cloth made of a spun bond layer and a melt blown layer; through-air nonwoven fabric cloth made of fibers, such as polyethylene fibers, polypropylene fibers, polyethylene terephthalate fibers, or the like; point bond nonwoven fabric cloth; and spun lace nonwoven fabric cloth. In addition, there may also be employed nonwoven fabric cloth which is formed from elastomer or copolymer and which possesses flexibility and an extensible characteristic. Moreover, in order to deodorize body's scent or the smell of body fluids, the nonwoven fabric cloth may be additionally provided with an adsorbent such as activated charcoal or zeolite; an antibacterial agent such as chitin, chitosan, or catechin; or a natural or synthetic fragrance such as limonene.

### [Second Embodiment]

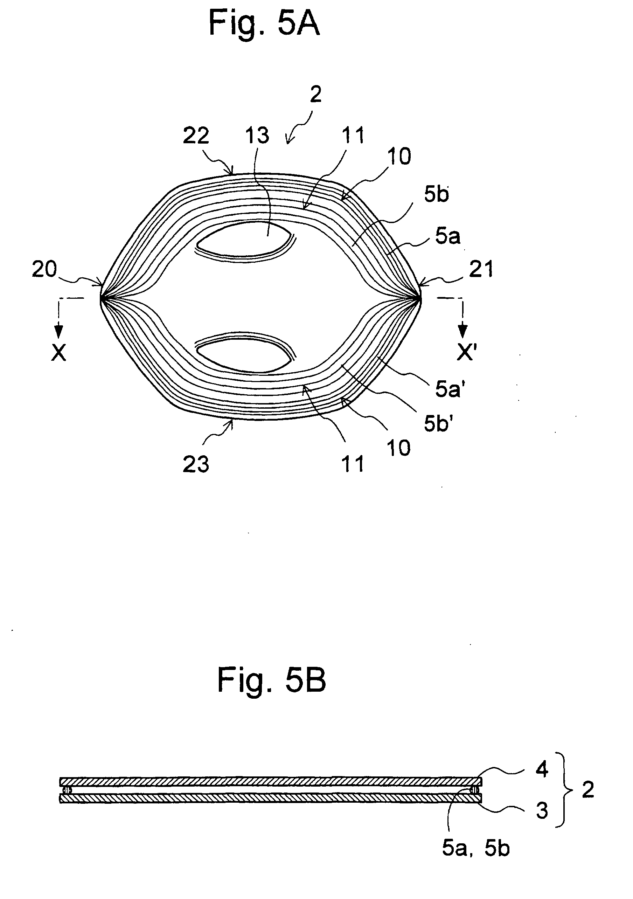

Fig. 5A is a view showing a developed state of an undergarment according to a second embodiment of the invention. Fig. 5B is a cross-sectional view taken along line X-X' shown in Fig. 5A. In the embodiment, those constituent elements which are the same as those employed in the first embodiment are assigned the same reference numerals, and their repeated explanations are omitted.

In the second embodiment, as shown in Fig. 3B, same as the first embodiment, the sheet member 2 is formed from an outer surface sheet 3, the inner surface sheet 4, and the elastic members 5a, 5a' and elastic member 5b, 5b' located in the waist opening section 10, the girth section 11 respectively are interposed between the sheets 3, 4 in a stretched manner. The elastic members 5a, 5a' of the waist opening section 10 are connected together so as to draw two sinusoidal curves and cross each other at one point. The elastic members 5a, 5a' are continuously provided in a stretched manner along the outer peripheral edge of the sheet member 2. in the other hand, the elastic members 5b, 5b' of the girth section 11 are also provided in a stretched manner such that the elastic members cross each other at one point. Even in this case, the elastic members 5a, 5a' of the waist opening section 10 are continuous with each other. Therefore, when the elastic members 5a, 5a' are subjected to contract, the outer peripheral edge of the sheet member 5a, 5a' is pulled, thereby forming a continuous ring-shaped waist opening section 10.

Even in the second embodiment, since the outer surface sheet 3 assumes a substantially-circular outer shape, the curves into which the elastic members 5a, 5a'are arranged assume a waveform pattern such as a substantially sinusoidal curve following the circular profile. However, the curves are not limited to such a waveform pattern. The curves may assume any shape, so long as the curves assume curves such as those shown in Figs. 3A to 3E or follow the outer shape of the outer layer sheet 3. For instance, when the outer layer sheet assumes a polygonal shape, the curves may assume a shape resembling a kinked graph matching the polygonal shape.

In addition to the manner of connecting together the elastic members 5a, 5a' located in the waist opening section 10, as shown in Fig. 3C the elastic members 5a, 5a' located in the waist opening section 10 may be connected together such that the elastic members draw waveform patterns, respectively, and such that a peak of one waveform comes into contact with a valley of the other waveform.

Alternatively, the elastic member 5a, 5a'located in the waist opening section 10 may differ in shape from that located in the girth section 11. The waveform of the elastic member 5a, 5a'located in the waist opening section 10 may form an intersection of a single point. In contrast, the elastic member 5b, 5b'located in the girth section 11 may be continuous such that a peak and a valley contact or cross each other (not shown) and vice versa. In this case, the elastic member 5a, 5a'located in the waist opening section 10 and the elastic member 5b, 5b' located in the girth section 11 become continuous with each other such that they cross each other at a single point.

However, as described in connection with the first embodiment, the elastic members 5a, 5a' located in the waist opening section 10 and the elastic members 5b, 5b' located in the girth section 11 are arranged consecutively and in a stretched manner and fixedly bonded to the sheet member 2. At this time, intersecting portions of the elastic members 5a, 5a', 5b, and 5b' remain non-bonded. When the continuous sheet member 2 is cut along the intersecting portions into disposable undergarments, the elastic members 5a, 5a' of waist opening section 10 and 5b, and 5b' of girth section 11 are arranged so as to cross each other may be subjected to contraction and be slightly separated from each other at locations where the elastic members are not fixedly bonded together. In this case, the elastic members 5a, 5a' of waist opening section 10 are partially discontinuous. However, the elastic members 5a, 5a' are laid along the outer peripheral edge of the sheet member 2 in a stretched and substantially continuous fashion. Hence, when the elastic members 5a, 5a' provided along the waist opening section 10 undergo contraction, they constitute a ring-shaped waist opening section 10. In this case, intersections disappear, and hence, the thickness of the elastic members can be reduced as compared with the thickness of the elastic members achieved when intersections are present. Thus, the wearer does not have unusual feeling.

In any of the manners of connecting the elastic members, the elastic members 5a, 5a' stretched in the waist opening section 10 are continuous to each other along the outer peripheral edge of the sheet member. Alternatively, the elastic members are substantially continuous with partially discontinuous portions. Hence, when the elastic members 5a, 5a' are subjected to contraction, the outer peripheral edges of the sheet member 2 are pulled, thereby constituting a continuous ring-shaped waist opening section 10. Consequently, an undergarment having no cemented section in the girth section 11 can be obtained.

### [Third Embodiment]

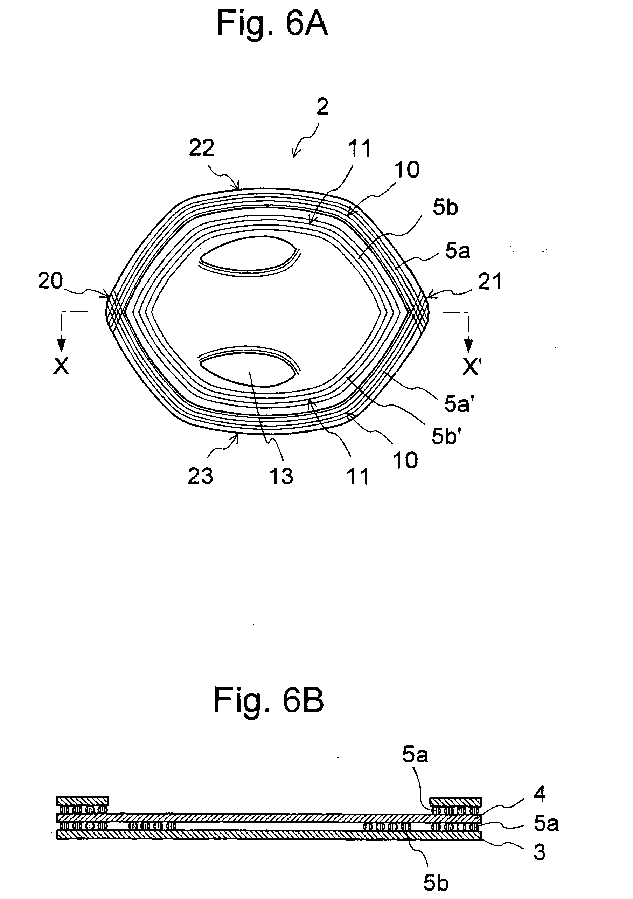

Fig. 6A is a developed plan view showing a developed state of a disposable undergarment according to a third embodiment of the invention. Fig. 6B is a cross-sectional view taken along line X-X' shown in Fig. 6A. In the embodiment, those constituent elements which are the same as those employed in the first embodiment are assigned the same reference numerals, and their repeated explanations are omitted.

Fig. 6A shows a developed state of a disposable undergarment according to the third embodiment of the invention. In relation to the disposable undergarment of the embodiment shown in Fig. 1, the elastic members 5a, 5a' to be laid along the waist opening section 10 in a stretched manner may be provided in a double layer as shown Fig. 6B. By means of a double layer structure, fastening the undergarment to the waist becomes tight, thereby improving fit. Consequently, even when having been subjected to vigorous workout, the undergarment becomes less likely to slip down.

Each embodiments described so far, the sheet members 2 assume a substantially circular shape although its shape is not limited to circular, and may assume a substantially polygonal shape of three (triangular) or more sides; for example, a substantially square shape, a substantially hexagonal shape, or a substantially octagonal shape. Moreover, the sheets may assume an oval shape. The shapes may be selected according to a wearer or an application, as required.

The sheet member 2 may be used in conjunction with a commercially-available liner. Moreover, the disposable undergarment may be used in lieu of a liner and along with a cloth undergarment. If the disposable undergarment is used as a lining for the fabric undergarment, the lining can prevent the undergarment from becoming stained with body fluids or the like.

In any of the embodiments, the disposable undergarment 1 of the embodiment has no cemented section. Hence, the undergarment cannot be torn along the cemented section after having been used. For this reason, not illustrated, means which enables occurrence of a rip is preferably provided in at least a part of the girth section 11. For example, the means which enables occurrence of a rip may be implemented by perforating the sheet member 2 or inflicting onto the sheet damage which enables occurrence of a rip, by means of heat fusing using ultrasonic waves.

As mentioned above, in the embodiments the disposable undergarment 1 of the invention is formed in the previously-described manner. Hence, the disposable undergarment has the following superior product characteristic. First, since the abdominal portion and the back portion of the sheet member 2 are not cemented together, the undergarment does not have a cemented section which extends from both leg opening sections 13 provided in the girth section to the waist opening section 10. For this reason, even when having worn the undergarment, the user does not feel any unusual feeling. Further, the undergarment does not have any cemented section, and hence the undergarment has good, superior appearance. When a garment, such as a pair of trousers or a skirt, is worn over the undergarment, the cemented sections do not appear through the garment, thereby failing to affect a silhouette of the garment.

Further, the disposable undergarment can be folded compact and carried conveniently. Even when being discarded after having been used, the undergarment can be discarded while being crumpled up.

In addition, the invention can be subjected to modifications within the scope of basic technical concept.

By reference to Fig. 7, there will now be described a method for manufacturing a disposable undergarment in which an elastic member located in a waist opening section 10 cross each other at one point, as well as an embodiment of the method (an embodiment provided in the form of a developed plan view shown in Figs. 5A). Since the material to be used for the disposable undergarment is identical with that described in connection with the embodiment, its repeated explanation is omitted.

As shown in Fig. 7, the disposable undergarment of the invention is continuously manufactured through a method, wherein the method comprising an continuous sheet member manufacturing step (I) of manufacturing an elastic-member-equipped continuous sheet member having an elastic member 106 by continuously placing an elastic member 102a, 102a' of the waist opening on a continuously-traveling outer surface sheet 101 in a stretched manner, a continuous sheet member shaping step (II) of forming leg opening sections 108 in areas of the continuous sheet member 106 where the elastic member is not provided and shaping the outer shape of the sheet member 106 by trimming, and an undergarment separation step (III) of separating pieces of disposable undergarments by cutting the continuous sheet member 2 in transverse direction.

Elastic-member-equipped continuous sheet member manufacturing process (I) is comprised of a continuous outer surface sheet 101 is unrolled while the direction of front and rear abdominal regions of the undergarment or the direction of right and left abdominal regions of the same is taken as the direction of flow, wherein elastic members 102a, 102a' of the waist opening section are provided in a stretched manner on the continuous outer surface sheet 101, such that the continuous inner surface sheet member 105 is joined together on the upper surface of elastic members 102a, 102a' which is provided in a stretched manner on the waist opening section.

A continuous outer surface sheet 101 is unrolled while the direction of front and rear abdominal regions of the undergarment or the direction of right and left abdominal regions of the same is taken as the direction of flow. Continuous elastic members 102a, 102a' of the waist opening section are provided in a stretched manner on the continuous outer surface sheet member 101 in the form of a waveform pattern by means of pivotal guides 103a, 103a' oriented in a direction orthogonal to the direction of flow of the continuous outer surface sheet member 101. A hot-melt adhesive is applied over the continuous outer surface sheet member 101 by means of an applicator 104. The hot-melt adhesive may be applied over the continuous outer surface sheet member 101 beforehand or applied to the elastic members 102a, 102a'. of the waist opening section Moreover, the adhesive to be applied to locations where the elastic members 102a, 102a' of the waist opening section are to be laid is preferably made larger in quantity than that applied to other locations.

At this time, as shown in Figs. 3B, the elastic members 102a, 102a' of the waist opening section stretched in the form of two waveforms are provided in a stretched manner such that all the elastic members cross each other at one point. However, the manner of stretching the elastic members is not limited to this manner. As shown in Fig. 3A, the elastic members may be laid in a stretched manner so as to cross each other. Alternatively, as shown in Fig. 3C and 3D, the two waveforms do not cross each other. The elastic members may be provided such that a valley of the waveform of the elastic member 102a of the waist opening section stretched by the pivotal guide 103a comes into contact at one point with or in close proximity to a peak of the elastic member 102a' of the waist opening section stretched by the pivotal guide 103a'.

Meanwhile, elastic members 102b, 102b' to be laid in a girth section are stretched discontinuously in the vicinity of the inside of the elastic members 102a, 102a' of the waist opening section by means of other pivotal guides (not shown). The elastic members may be stretched in same manner as 102a, 102a' so as to cross each other at one point or at a plurality of points or to come into contact with or in proximity to each other at one point while being continuously fed in the same manner as is the elastic member of the waist opening section.

Moreover, the elastic members 102a, 102a' to be provided in the waist opening section and the elastic members 102b, 102b' to be provided in the girth section, are made different from each other in terms of shape of waveforms wherein the elastic members may be stretched by means of different methods such that the elastic members 102a, 102a'to be provided in the waist opening section cross each other at one point and such that peaks and valleys of the waveform of the elastic member102b, 102b' to be provided in the girth section come into contact with each other or in close proximity to each other.

In any event, the manner of connecting the elastic members 102a, 102a'is not limited to the above-described method. Any method may be employed, so long as the elastic members 102a, 102a' to be stretched in the waist opening section are stretched continuously along the outer peripheral edge of the continuous outer surface sheet member 101.

Although not illustrated, it is better to stretch elastic members to be stretched in the side edge portions of the leg opening sections 108 simultaneously with stretching of the elastic members 102a, 102a' 102b, 102b' in the waist opening section and the girth section.

A continuous inner surface sheet member 105 is continuously unrolled while the direction of front and rear abdominal regions of the undergarment or the direction of right and left abdominal regions of the same is taken as the direction of flow. The continuous outer surface sheet member 101 and the continuous inner surface sheet member 105 are cemented together such that the sheet members 101, 105 are sandwiched between the elastic members 102a, 102a' 102b, 102b', thereby forming a continuously-traveling sheet member 106.

When the continuous inner surface sheet member 105 is not used, processes wherein the continuous inner surface sheet member 105 is joined together on the upper surface of elastic members 102a, 102a' which is provided in a stretched manner on the waist opening section, may be omitted.

Next, the continuous sheet member shaping step (II), wherein a continuously-traveling sheet member 106 is trimmed by means of a roll cutter 107, is shaped into a continuous member formed from substantially-circular continued members, and leg opening sections 108 are formed and further shaped into processed continuous sheet member 110. When the leg opening sections 108 are formed while the direction of front and rear abdominal regions is taken as the direction of flow, the leg opening sections 108 are formed at symmetrical positions while the axis of symmetry of the processed continuous sheet member 110 (direction in which the sheet member 110 is to flow) is taken as the axis of symmetry, as in the case of the sheet member shaping process shown in Fig. 7. Although not illustrated, when the processed continuous sheet member 110 is formed while the direction of right and left abdominal regions is taken as the direction of flow, the only requirement is to form the leg opening sections 108 at symmetrical positions while the direction orthogonal to the axis of symmetry of the processed continuous sheet member 110 (direction orthogonal to the direction of flow of the processed continuous sheet member 110) is taken as the axis of symmetry.

Next, an undergarment separation step (III) involves the thus-shaped processed sheet member 110 which is cut in the transverse direction thereof at positions where the elastic members 102a, 102a' of the waist opening section 10 crossing each other, thereby producing pieces of individual sheet member 2.

Waist opening section, created on the individual sheet member 2 by means of shrinking elastic member 102a, 102a' of the waist opening section, is formed into an undergarment.

According to the manufacturing method of the invention, a sheet member is formed while elastic members located at the waist opening section are stretched in the direction of front and rear abdominal regions or the direction of right and left abdominal regions so as to draw two waveform patterns, such that the elastic members constituting the waveform patterns cross each other at one point or a plurality of points or come into contact with or in close proximity to each other at one point as described in Fig.3A through 3E. Hence, the elastic members stretched along the outer peripheral edge of the continuous sheet member is stretched such that two waveforms become continuous. When the elastic-member equipped continuous sheet member which has been formed continuously and has an elastic member is cut into pieces of sheet members, the elastic members undergo contraction, thereby forming a ring-shaped waist opening section. Consequently, there is obviated a necessity for sewing a sheet member at a front abdominal section, a rear abdominal section, and a crotch section, which has conventionally been required for manufacturing a conventional undergarment. Therefore, processes for sewing the front abdominal section, the rear abdominal section, and the crotch section can be omitted, thereby improving productivity.

Needless to say, the disposable undergarments and the method for manufacturing the same are not limited to the previously-described embodiments and the previously-described manner of embodying the invention. The disposable undergarments of the invention are susceptible to various modifications without departing from the scope of the invention. In connection with the manner of implementing the manufacturing method of the invention, various methods can be selected without departing from the scope of the invention.

For instance, according to the manufacturing method, a conventionally-known method, such as an ultrasonic bonding method or a crimping method, can be utilized as the method for cementing together constituent materials other than the elastic members, in addition to a hot melt adhesive or a heat sealer. A conventionally-known applicator, such as a spiral spray gun suitable for spraying an adhesive on a plane, a spray gun of curtain spray to which a melt blown method is applied, or a bead nozzle method suitable for applying an adhesive on a line, can be utilized as a device for applying an adhesive. Moreover, in addition to the roll cutter, a conventionally-known cutter such as a water pressure cutter can be utilized for shaping a sheet member. Furthermore, a mode of implementation in which a sheet member is formed from only an outer surface sheet may be employed as a mode for implementing a disposable undergarment.

As has been described, in relation to the disposable undergarment of the invention, the elastic members to be placed in the outer peripheral waist opening section of the sheet member are placed continuously while having been stretched beforehand. Hence, when the elastic members undergo contraction, the outer peripheral edge of the sheet member is pulled, thereby forming a ring-shaped waist opening section and become undergarment. Since the right and left abdominal regions are continuous, the undergarment does not have any cemented section, which would otherwise be formed by sewing together a front abdominal section, a rear abdominal section, and a crotch section of a conventional disposable undergarment. Therefore, the wearer does not have any unusual feeling in the neighborhood of the legs. Further, since the undergarment has no cemented section, the undergarment has good looking and superior appearance.

Since the undergarment does not have any cemented section, the undergarment does not become voluminous. Hence, when folded, the undergarment becomes very compact. The undergarment is very convenient for carrying during outdoor activities s or travel. In addition, since the undergarment can be folded small for discarding after use, the thus-discarded undergarment requires only small quantity when handled as waste.

Since the undergarment has no cemented section, the cemented section does not appear through a garment such as a pair of trousers or a skirt when the garment is worn over the undergarment, and hence a silhouette of the garment is not affected.

According to the manufacturing method of the invention, stretching of the elastic members to be placed in the waist opening section and formation of the leg opening sections can be effected in the form of a continuous flow in a single direction. Hence, the undergarments can be produced at high speed without involvement of a drop in production speed and hence are superior in productivity.

When the continuously-formed sheet member is cut into pieces, elastic members provided on the sheet member are continuous. Hence, there is formed an undergarment in which a ring-shaped continuous waist opening section is formed as a result of the elastic member having undergone contraction. Hence, sewing processes which have been required for manufacturing conventional undergarments become obviated, thereby improving productivity.

## Claims

1. A disposable undergarment having a sheet member to constitute an exterior, an elastic member for causing a portion of the sheet member to contract, and a pair of leg opening sections through which a wearer's legs penetrate, wherein
the sheet member is formed from a material which is seamless with respect to a circumferential direction of the leg when the sheet member is worn.

2. The disposable undergarment according to claim 1, wherein the elastic member is provided along an substantially outer peripheral edge of the sheet member in a previously-stretched manner, the pair of leg opening sections are formed in the vicinity of the inside of the elastic member and at positions outside the center of the sheet member; and wherein a waist opening section is formed as a result of the elastic member stretched in the sheet member undergoing contraction.

3. The disposable undergarment according to claim 1 or 2, wherein the sheet member is formed from an outer surface sheet and an inner surface sheet which is substantially identical in size and shape with the outer surface sheet, the elastic member is interposed between the outer and inner surface sheets and along an outer peripheral edge of the sheet member in a previously-stretched manner, the pair of leg opening sections are provided in the vicinity of the inside of the elastic member and at positions outside the center of the sheet member; and wherein a waist opening section is formed as a result of the elastic member stretched on the sheet member having undergone contraction.

4. The disposable undergarment according to any one of claims 1 through 3, wherein a plurality of the elastic members are provided around the waist opening section and a girth in a previously-stretched state.

5. The disposable undergarment according to claim 4, wherein the elastic members provided in the waist opening section cross each other in either front and rear abdominal regions or right and left abdominal regions.

6. The disposable undergarment according to claim 4, wherein the elastic members provided in the waist opening section cross or overlap each other at one point each in either front and rear abdominal regions or right and left abdominal regions.

7. The disposable undergarment according to any one of claims 1 through 6, wherein the elastic member is rubber yarn, flat rubber, or a ribbon-shaped elastic body.

8. The disposable undergarment according to any one of claims 1 through 7, wherein the sheet member is hydrophobic and gas-permeable nonwoven fabric cloth.

9. The disposable undergarment according to claim 8, wherein the sheet member is nonwoven fabric cloth provided with a deodorant.

10. The disposable undergarment according to claim 8, wherein the sheet member is nonwoven fabric cloth provided with a fragrance.

11. The disposable undergarment according to any one of claims 8 through 10, wherein the nonwoven fabric cloth is nonwoven fabric cloth selected from the group consisting of continuous polyolefine-based resin fibers.

12. The disposable undergarment according to any one of claims 8 through 11, wherein a crotch section of the sheet member is liquid-impervious and moisture-permeable.

13. The disposable undergarment according to any one of claims 8 through 11, wherein the undergarment is to be used for a sanitary product having a mechanical fastener or for attaching an auxiliary product of the sanitary product.

14. The disposable undergarment according to any one of claims 1 through 11, wherein the undergarment is to be used as a lining of an ordinary underwear, to thereby prevent staining of the underwear.

15. A method for manufacturing a disposable undergarment, comprising at least an elastic-member-equipped continuous sheet member manufacturing step of manufacturing a continuous sheet member having an elastic member by continuously placing an elastic member on a continuously-traveling outer surface sheet in a stretched manner, a continuous sheet member shaping step of forming leg opening sections in areas of the continuous sheet member where the elastic member is not provided and shaping the outer shape of the sheet member by trimming and an undergarment separation step of separating pieces of disposable undergarments by cutting the continuous sheet member in transverse direction.
